(19) Europäisches Patentamt
European Patent Office
Office européen des brevets

(11) Publication number: **0 194 833**
**A2**

(12) # EUROPEAN PATENT APPLICATION

(21) Application number: 86301686.1

(22) Date of filing: 10.03.86

(51) Int. Cl.[4]: C 07 H 17/08
A 61 K 31/70

(30) Priority: 12.03.85 GB 8506380

(43) Date of publication of application:
17.09.86 Bulletin 86/38

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: Hunt, Eric
10 The Avenue Brockham
Betchworth Surrey RH3 7EN(GB)

(74) Representative: Dayneswood, Trevor et al,
Beecham Pharmaceuticals Patent and Trade Mark
Department Biosciences Research Centre Great Burgh
Yew Tree Bottom Road
Epsom Surrey KT18 5XQ(GB)

(54) **Erythromycin derivatives.**

(57) 6-ether derivatives of erythromycin 9-oximes and 9-oxime-ethers are novel antibacterially active compounds, and can be prepared by alkylation of the 6-hydroxy group.

EP 0 194 833 A2

TITLE MODIFIED
0194833
see front page

B1786

## CHEMICAL COMPOUNDS

The present invention relates to novel chemical compounds, their preparation and their use, and in particular to a novel class of erythromycin derivatives. These compounds have antibacterial properties, in particular against Gram-positive bacteria but also against some Gram-negative bacteria, and they are therefore of use in the treatment of bacterial infections in humans and animals caused by a wide range of organisms.

Erythromycin was first described in US 2 653 899 (R.L. Bunch *et al*; Eli Lilly). The structure of erythromycins can be represented as follows:

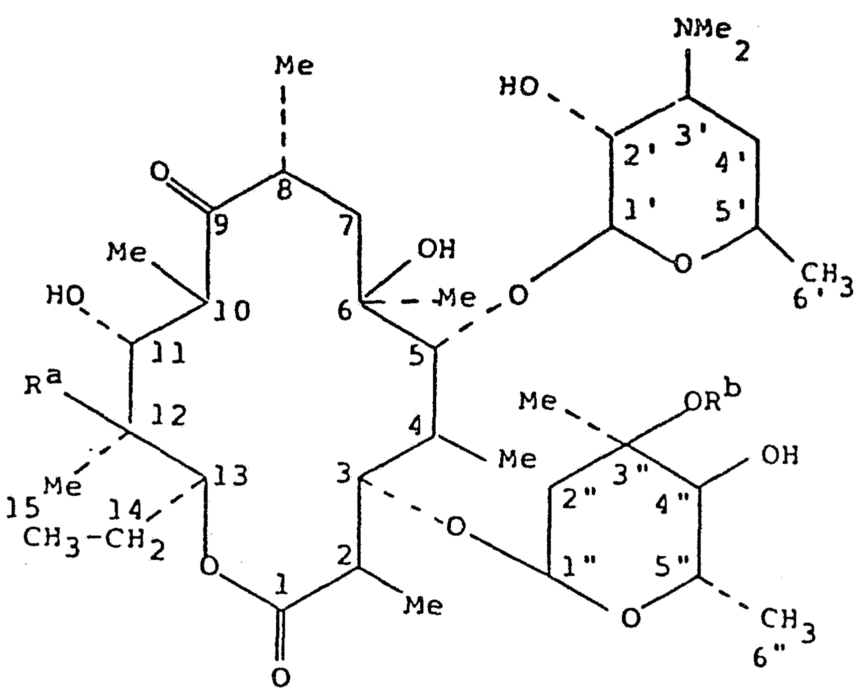

in which

$R^a$ denotes hydrogen or hydroxy and

$R^b$ denotes hydrogen or methyl.

The basic erythromycin structure comprises:

(i) a 14-membered lactone ring, referred to as the erythronolide ring, numbered with unprimed digits as shown in the above formula,

(ii) a first sugar ring, known as the desosamine ring, numbered with single-primed digits, and

(iii) a second sugar ring, known as the cladinose ring, numbered with double-primed digits.

The erythronolide ring can exist in two forms:

erythronolide A (in which $R^a$ = OH)

erythronolide B (in which $R^a$ = H).

The four main naturally occurring erythromycins are as follows:

| Erythromycin | $R^a$ | $R^b$ |
|---|---|---|
| A | OH | $CH_3$ |
| B | H | $CH_3$ |
| C | OH | H |
| D | H | H |

of which erythromycin A is by far the most important.

Erythromycins, and in particular erythromycin A, are antibiotics widely employed clinically in the treatment of infections caused by Gram-positive and some Gram-negative bacteria. A major drawback of erythromycins is their poor acid stability, resulting in poor and erratic oral absorption.

Numerous attempts have been made to modify erythromycin to produce derivatives having improved acid stability without loss of the antibacterial activity.

(9S)-9-Dihydroerythromycin A (which carries a 9-hydroxy group in place of the 9-keto group) has been described, but has poor antibacterial activity (P.F. Wiley et al, J. Amer. Chem. Soc., 1955, 77, 3676-3677; M.V. Sigal et al, ibid, 1956, 78, 388-395; and T. Glabski et al, Roczniki Chem., 1976, 50, 1281). Erythromycylamine and erythromycin oxime (in which the 9-keto group is replaced, respectively, by an amino or oxime group), as well as various N-substituted derivatives of erythromycylamine have also been described (GB 1 100 504 (Pliva Pharmaceutical); E.H. Massey et al, Tetrahedron Letters, 1970, No. 2, 157-160; and G.H. Timms et al, ibid, 1971, No. 2, 195-198), as have various erythromycin oxime ethers (US 3 681 326 (A.M. Von Esch; Abbott Laboratories); US 3 869 445 and US 4 063 014 (both R. Hallas et al; Abbott Laboratories); and US 4 349 545 (S. Gouin d'Ambrieres; Roussel-Uclaf); and Antimicrobial agents and chemotherapy, 1974, 6, 479).

Certain aldehyde-erythromycylamine condensation products with a 9-N,6-O- or 9-N,11-O-cyclic substituent have previously been disclosed (US 4 048 306 (R. Maier et al; Boehringer Ingelheim GmbH)).

4''-Deoxy-11-O-methylthiomethyl-4''-oxo-erythromycin B and its conversion to (i) 4''-deoxy-9,11-O-(optionally substituted)methylene-4''-oxo-erythromycin B 6,9-hemiacetal and the corresponding 4''-*epi*-hydroxy, 2',4''-O-diacetyl-4''-*epi*, and 4''-O-acetyl-4''-*epi* derivatives, and (ii) 4''-deoxy-4''-oxo-, 4''-O-acetyl-4''-*epi*-, and 4''-*epi*-erythromycin B; as well as 4''-O-formyl-11-O-methylthiomethyl-erythromycin B and its conversion to 11-O-methylthiomethyl-erythromycin B, 9,11-O-methylene-erythromycin B 6,9-hemiacetal, 11-O-methyl-erythromycin B and 11-O-*n*-butyl-erythromycin B; and also 4''-deoxy-4''-oxo-erythromycin A are described in US 3 842 069, US 3 884 903 and US 3 884 904 (all P.H. Jones *et al*; Abbott Laboratories).

4''-Deoxy-4''-amino-erythromycin A, 4''-deoxy-4''-amino-erythromycin A 6, 9-hemiketal, and 4''-deoxy-4''-oxo-erythromycin A 6,9-hemiketal, as well as corresponding 11-O-acetyl and 11,12-cyclic carbonate derivatives, and also 4''-deoxy-4''-amino-erythromycin B and 4''-deoxy-4''-oxo-erythromycin A 4''-O-oxime or 4''-O-acetyloxime, are described in US 4 150 220 (F.C. Sciavolino; Pfizer).

An 11,12-cyclic carbonate of 9-dihydroerythromycin has also been described in T. Glabski *et al*; *Roczniki Chem.*, 1976, *50*, 1281 and 9-dihydro-11,12-O-isopropylidene -erythromycin A and the corresponding 4''-*epi* compound have been described in US 4 382 086 (F.C. Sciavolino *et al*; Pfizer).

6-O-Methyl-, 6,11-di-O-methyl-, 11-O-methyl- and 11-O-ethyl-erythromycin A, and also 6-O-methyl-6,4''-di-O-methyl-, and 6,11,4''-tri-O-methyl-erythromycin B are described in EP 0 041 355 A1, EP 0

080 818 A1 and EP 0 080 819 A1 (all Taisho Pharmaceutical). 6-O-methyl-erythromycin A derivatives and their preparation are also described in EP 0 158 467 A2 (Taisho; priority 06 April 1984, filed 22 March 1985 (subsequent to the priority date of the present application), published 16 October 1985).

The erythromycin derivatives according to the present invention in general possess improved acid stability ascompared with erythromycin A while retaining good antibacterial activity.

The present invention provides antibacterially active 6-ether derivatives of erythromycin oxime, and corresponding 9-(optionally substituted)amino, 9-imino, and 9-oxime ether compounds.

In particular, the present invention provides a compound of the general formula I or a pharmaceutically acceptable ester or acid addition salt thereof:

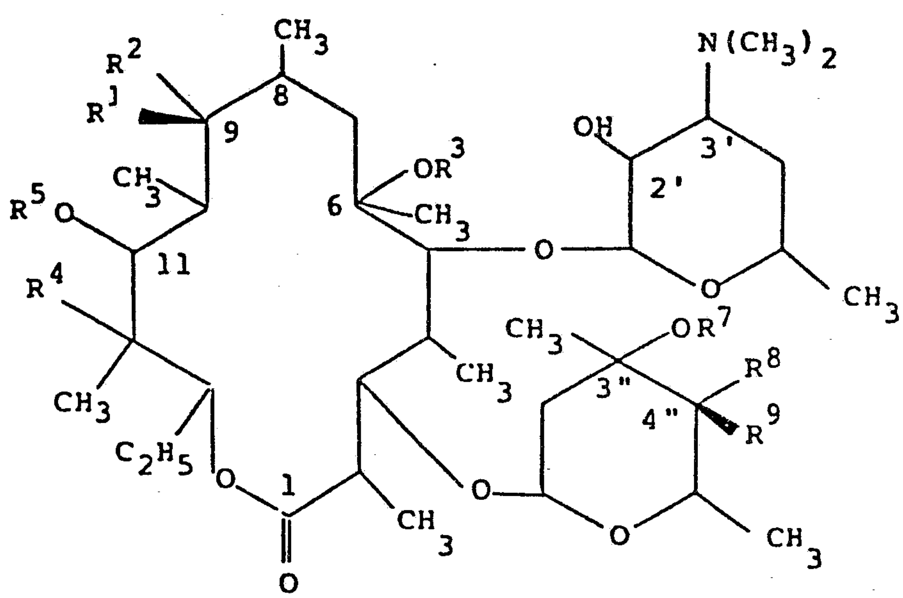

I

wherein

one of $R^1$ and $R^2$ denotes hydrogen and the other of $R^1$ and $R^2$ denotes an amino group or a substituted amino group, or

$R^1$ and $R^2$ together denote an oxime group, an oxime ether group, or an imino group;

$R^3$ denotes an unsubstituted or substituted alkyl group;

$R^4$ denotes hydrogen or hydroxy;

$R^5$ denotes hydrogen, an alkyl group, or a group of the formula $-CHR^{10}SR^{11}$;

$R^7$ denotes hydrogen or methyl;

one of $R^8$ and $R^9$ denotes hydrogen, hydroxy, alkoxy, alkanoyloxy, amino, substituted amino, or a group of the formula $R^{12}-SO_2-O-$, and the other of $R^8$ and $R^9$ denotes hydrogen, or

$R^8$ and $R^9$ together denote an oxo group, an oxime group, or a substituted oxime group;

$R^{10}$ denotes hydrogen or an alkyl group;

$R^{11}$ denotes an alkyl group; and

$R^{12}$ denotes an organic group.

The term 'hydrocarbon' as used herein includes groups having up to 18 carbon atoms, suitably up to 10 carbon atoms, conveniently up to 6 carbon atoms. Suitable hydrocarbon groups include $(C_{1-6})$alkyl, $(C_{2-6})$alkenyl, $(C_{2-6})$alkynyl, $(C_{3-7})$cycloalkyl, aryl,

$(C_{3-7})$cycloalkyl$(C_{1-6})$alkyl, aryl$(C_{1-6})$alkyl, $(C_{1-6})$alkyl$(C_{3-7})$cycloalkyl, and $(C_{1-6})$alkylaryl.

Examples of suitable optional substituents for the above-mentioned hydrocarbon groups include, heterocylyl, amino, $(C_{1-6})$alkanoylamino, (mono, di, or tri)-$(C_{1-6})$alkylamino, hydroxy, $(C_{1-6})$alkoxy, mercapto, $(C_{1-6})$alkylthio, heterocyclylthio, arylthio, sulphamoyl, carbamoyl, amidino, guanidino, nitro, halogen (for example chloro, bromo, fluoro), carboxy and salts and esters thereof acyl and acyloxy groups.

Any alkyl group or moiety referred to herein may be straight or branched, unsubstituted or substituted, and may contain, for example, up to 12 carbon atoms, suitably up to 6 carbon atoms. In particular, the alkyl group or moiety may be an unsubstituted or substituted methyl, ethyl, n-propyl, iso-propyl, n-butyl, sec-butyl, isobutyl or tert-butyl group. Examples of suitable optional substitutents for any such alkyl group or moiety include the above-listed substitutents for hydrocarbon groups, and also the above-listed non-alkyl hydrocarbon groups, for example $(C_{2-6})$alkenyl and aryl groups.

The term 'aryl' as used herein includes phenyl and naphthyl, which may be unsubstituted or substituted by up to five, preferably up to three, groups selected from the above-listed substituents for hydrocarbon groups, and the above-listed hydrocarbon groups, including for example, substituents selected from halogen, $(C_{1-6})$alkyl, phenyl, $(C_{1-6})$alkoxy, halo$(C_{1-6})$alkyl, hydroxy, amino, nitro, carboxy, $(C_{1-6})$alkoxycarbonyl, $(C_{1-6})$alkoxycarbonyl$(C_{1-6})$alkyl, $(C_{1-6})$alkanoyloxy, and $(C_{1-6})$alkanonyl groups.

The term 'acyl' as used herein includes unsubstituted and substituted hydrocarbon-carbonyl and hydrocarbonoxy-carbonyl groups, including, for example, unsubstituted and substituted alkanoyl, cycloalkylcarbonyl, arylcarbonyl, alkoxycarbonyl, aryloxycarbonyl, and heterocyclylcarbonyl groups. The term 'acyloxy' is used analogously.

The terms 'heterocyclyl' and 'heterocyclic' as used herein include aromatic and non-aromatic, single and fused, rings suitably containing up to four hetero-atoms in each ring selected from oxygen, nitrogen and sulphur, which rings may be unsubstituted or substituted by, for example, up to three groups selected from halogen, $(C_{1-6})$alkyl, $(C_{1-6})$alkoxy, halo-$(C_{1-6})$alkyl, hydroxy, amino, carboxy, carboxy salts, carboxy esters, $(C_{1-6})$alkoxycarbonyl, $(C_{1-6})$alkoxy-carbonyl$(C_{1-6})$alkyl, aryl, and oxo groups. Each heterocyclic ring suitably has from 4 to 7, preferably 5 or 6, ring atoms. A fused heterocyclic ring system may include carbocyclic rings and need include only one heterocyclic ring.

The term 'heteroaryl' as used herein means an aromatic heterocyclic ring or ring system, suitably having 5 or 6 ring atoms in each ring.

In one group of compounds of the general formula I, $R^1$ and $R^2$ together denote an oxime group (also referred to as a hydroxyimino group, =NOH) or an oxime ether group. Such compounds may be referred to as erythromycin oxime or oxime-ether derivatives. In a second group of compounds of the general formula I, $R^1$ and $R^2$ together denote an imino group, and such compounds may be referred to as erythromycin imines.

In a third group of compounds of the general formula I, one of $R^1$ and $R^2$ denotes an amino group or a substituted amino group, and the other of $R^1$ and $R^2$ denotes a hydrogen atom; such compounds may be referred to as erythromycylamines.

In the case of the erythromycin oxime and oxime-ether derivatives according to the invention, $R^1$ and $R^2$ may together denote a group of the formula II:

$$=N \sim\sim\sim O-R^{13} \qquad \text{II}$$

in which $R^{13}$ denotes hydrogen or an unsubstituted or substituted hydrocarbon group. Examples of suitable groups denoted by $R^{13}$ include unsubstituted and substituted alkyl, cycloalkyl and aryl (preferably phenyl) groups. Examples of unsubstituted alkyl groups $R^{13}$ include methyl, ethyl, n-propyl, isopropyl, n-butyl, sec-butyl, and tert-butyl groups. Examples of substituted alkyl groups $R^{13}$ include aralkyl (especially benzyl), alkoxyalkyl, alkenyloxyalkyl, alkynyloxyalkyl, aryloxyalkyl, arylalkoxyalkyl, alkoxyalkoxyalkyl (for example, β-methoxyethoxymethyl), alkylthioalkyl, alkenylthioalkyl, alkynylthioalkyl, arylthioalkyl, aralkylthioalkyl, haloalkyl, formylalkyl, carboxyalkyl and salts and esters thereof, thiocyanotoalkyl, cyanoalkyl, acylalkyl, carbomoylalkyl, and aminoalkyl groups; each of the said alkyl, alkenyl and alkynyl moieties suitably having up to 6 carbon atoms; each of the said thio derivatives optionally being oxidised to the corresponding sulphoxide or sulphone derivative; and the said amino moiety of the said aminoalkyl groups suitably being of the formula III:

$$\begin{array}{c} R^{14} \\ | \\ -N \\ | \\ R^{15} \end{array} \qquad\qquad III$$

in which each of $R^{14}$ and $R^{15}$, which may be identical or different, denotes hydrogen or an unsubstituted or substituted hydrocarbon group, advantageously an alkyl group, preferably having from 1 to 6 carbon atoms, or $R^{14}$ and $R^{15}$ and the nitrogen atom to which they are attached together denote an unsubstituted or substituted, unsaturated or saturated heterocyclic ring, optionally containing one or more heteroatoms additional to the said nitrogen atom, each of $R^{14}$ and $R^{15}$ preferably denoting a hydrogen atom.

Erythromycin oximes and oxime-ethers having 9-substituents of the type described above have been described in, for example, GB 1 100 504, E.H. Massey et al, G.H. Timms et al, US 3 681 326, US 3 869 445, US 4 063 014 and US 4 349 545, all op. cit..

The erythromycin oxime and oxime-ether derivatives according to the invention can exist in two geometric isomeric forms about the C=N double bond at the 9-position, as indicated by the wavy line in formula II above, namely the E-form and the Z-form. The E-form is generally preferred.

In the case of the erythromycin imine derivatives according to the invention, $R^1$ and $R^2$ together denote a group of the formula IV:

$$=N-H \qquad\qquad IV$$

Erythromycin imine has been described, for example, in G.H. Timms et al, op. cit..

In the case of the erythromycylamine derivatives according to the invention, one of $R^1$ and $R^2$ denotes hydrogen and the other of $R^1$ and $R^2$ may denote a group of the formula III above, in which $R^{14}$ and $R^{15}$ are defined as above. Suitably each of $R^{14}$ and $R^{15}$ denotes a hydrogen atom. Erythromycylamine and derivatives thereof have, for example, been described in GB 1 100 504, E.H. Massey et al and G.H. Timms et al, all op. cit..

The erythromycylamine derivatives according to the invention can exist in two isomeric forms at the 9-position, namely the (9R)-form, in which $R^1$ denotes hydrogen and $R^2$ denotes the optionally substituted amino group, and the (9S)-form, in which $R^1$ denotes the optionally substituted amino group and $R^2$ denotes hydrogen. The (9S)-isomer is preferred.

The erythromycin derivatives according to the invention are characterised by a 6-ether group, denoted as $-OR^3$ in the general formula I. $R^3$ denotes an unsubstituted or substituted alkyl group, which may advantageously be an unsubstituted or substituted primary alkyl, group (i.e. an alkyl group in which the α-carbon atom carries at least two hydrogen atoms). Suitably, the alkyl group may be a lower alkyl group, preferably a $(C_{1-6})$alkyl group, for example a methyl or ethyl group. Examples of substituted alkyl groups denoted by $R^3$ include aralkyl groups, for example a benzyl group, and alkenylalkyl groups, for example an allyl group. Suitably $R^3$ denotes an unsubstituted alkyl group, preferably a lower alkyl group, and especially a methyl group.

Examples of suitable substitutents for an alkyl group $R^3$ include, in particular, alkenyl (for example, ethenyl), aryl (for example, phenyl), alkoxy, alkoxyalkoxy, aryloxy, hydroxy, amino, substituted amino (for example, monoalkylamino and dialkylamino), carboxy, esterified carboxy (for example, alkoxycarbonyl), acyloxy (wherein 'acyl' means organic-carbonyl) (for example, alkanoyloxy), carbamoyl ($H_2N-C(=O)-$), and substituted carbamoyl (for example, N-alkylcarbamoyl and N,N-dialkylcarbamoyl) groups. Any aryl or alkyl moiety in such substituents may itself be substituted by, for example, an alkyl or aryl group or one of the listed substituents, and any alkyl moiety advantageously contains not more than 6, preferably not more than 4, carbon atoms. An example of a substituent in which an alkyl moiety is itself substituted is an alkoxyalkoxy substituent.

In the compounds of the general formula I, the 12-substituent denoted by $R^4$ is preferably a hydroxy group as in the erythronolide A ring, or, in other words, the compounds of the general formula I are preferably derivatives of erythromycin A. Alternatively, however, the present compounds may be derivatives of erythromycin B, in which case $R^3$ denotes a hydrogen atom, or of another naturally occurring erythromycin.

The 11-position of the erythronolide ring may carry a hydroxy group or an etherified hydroxy group, $-OR^5$, in which $R^5$ denotes hydrogen, alkyl, or a group of the formula V:

$$-CHR^{10}SR^{11} \qquad V$$

in which $R^{10}$ and $R^{11}$ are defined as above. Suitably, the 11-position carries a hydroxy group, as in naturally-occuring erythromycins, in which case $R^5$ denotes hydrogen.

In the event that the 11-position carries an etherified hydroxy group, $R^5$ suitably denotes an alkyl group, advantageously a $(C_{1-6})$alkyl group, preferably a methyl group. 11-O-Alkyl-erythromycin derivatives have been described in US 3 884 904, EP 0 041 355 A1, EP 0 080 818 A1, and EP 0 080 819 A1, all op. cit.. In the event that $R^5$ denotes a group of the formula V above, $R^{10}$ suitably denotes a hydrogen atom or a $(C_{1-6})$alkyl group and $R^{11}$ suitably denotes a $(C_{1-6})$alkyl group, preferably a methyl group. An example of a group $R^5$ of the formula V is a methylthiomethyl group. 11-O-Alkylthioalkyl-erythromycin derivatives have been described in US 3 842 069 and US 3 884 904, both op. cit..

The $-OR^7$ group in the 3''-position of the cladinose ring may be a hydroxy group or a methoxy group. Preferably, $R^7$ denotes a methyl group as in erythromycin A and B.

The 4''-position of the cladinose ring may suitably carry a hydroxy group as in erythromycin A and B ($R^8$ = H; $R^9$ = OH). Various modifications of the 4''-position of the cladinose ring have previously been described and those modifications may be incorporated in the compounds according to the present invention:

(i) 4''-deoxy-4''-oxo derivatives ($R^8 + R^9$ = O=) are described in US 3 842 069, US 3 884 903 and US 4 150 220, all op. cit.;

(ii) 4''-*epi*-hydroxy derivatives ($R^8$ = OH; $R^9$ = H) and 4''-deoxy-4''-alkanoyloxy-4''-*epi* derivatives ($R^8$ = alkanoyloxy, especially $CH_3COO-$; $R^9$ = H) are described in US 3 884 903, *op. cit.*;

(iii) 4''-*O*-alkyl derivatives ($R^8$ or $R^9$ = alkoxy, especially methoxy; the other of $R^8$ and $R^9$ = H) are described in EP 0 080 818 A1, *op. cit.*;

(iv) 4''-deoxy-4''-amino derivatives ($R^8$ or $R^9$ = amino or substituted amino; the other of $R^8$ and $R^9$ = H) are described in US 4 150 220, *op. cit.*;

(v) 4''-deoxy-4''-oxime derivatives ($R^8$ + $R^9$ = oxime (=N-OH) or substituted oxime, especially acetyloxime ($=N-O-CO-CH_3$)) are also described in US 4 150 220, *op cit.*;

(vi) 4''-*O*-sulphonyl derivatives ($R^8$ = H, $R^9$ = $R^{12}-SO_2-O-$) are described in US 3 836 519, US 3 869 445 and US 4 063 014 (all R. Hallas *et al*; Abbott Laboratories); and

(vii) 4''-deoxy derivatives ($R^8$ = $R^9$ = H) are described in JP 58-049396 (Toyo Jozo KK).

In the 4''-deoxy-4''-(substituted amino) derivatives, the substituted amino group $R^8$ or $R^9$ may suitably be a group of the formula

$$-NHCOR^{16} \quad \text{or} \quad -NHSO_2R^{16}$$

in which $R^{16}$ denotes a hydrocarbon group.

In the 4''-O-sulphonyl derivatives, in which $R^8$ or $R^9$ denotes a sulphonyloxy group of the formula

$R^{12}-SO_2-O-$,

the organic group $R^{12}$ may suitably be an unsubstituted or substituted hydrocarbon, oxahydrocarbon, thiahydrocarbon or azahydrocarbon group, more especially an alkyl, alkenyl, unsubstituted or substituted aryl (especially phenyl, nitrophenyl, halophenyl or alkylphenyl), unsubstituted or substituted aralkyl (especially benzyl, nitrobenzyl, halobenzyl or alkylbenzyl), unsubstituted or substituted aryloxyalkyl (especially phenoxyalkyl, nitrophenoxyalkyl, halophenoxyalkyl or alkylphenoxyalkyl), or substituted ethyl (especially $R^{17}-CH_2-CH_2-$, wherein $R^{17}$ is defined as below) group.

Examples of groups $R^{17}$ in the 4''-substituent

$R^{17}-CH_2-CH_2-SO_2-O-$

include amino, substituted amino, carbamoyl, substituted carbamoyl, sulphamoyl, substituted sulphamoyl, substituted ureido, substituted thioureido, alkoxy, alkythio, optionally substituted aryloxy, optionally substituted arylthio, optionally substituted benzyloxy, optionally substituted benzylthio, substituted suphonyl, substituted sulphinyl, substituted alkyl, substituted alkanoyl, substituted cyano, and other groups more specifically described in US 3 869 445 and US 4 063 014, op. cit.

Preferably, $R^{12}$ denotes a hydrocarbon group, particularly a $(C_{1-6})$alkyl group, especially a methyl group.

The present invention includes pharmaceutically acceptable esters, especially *in vivo* hydrolysable

esters, of the compounds of the general formula I. The esters may be formed at any hydroxy group in the compounds of the general formula I, but usually the ester will be formed at the 2'-hydroxy group of the desosamine ring, thus giving a 2'-*O*-acyl derivative of the type described in US 2 862 921 (R.E. Booth *et al*; Upjohn Co.), US 2 993 833 (V.C. Stephens; Eli Lilly), US 3 836 519, US 3 842 069, US 3 869 445, US 3 884 903, US 3 884 904 and US 4 150 220, all *op. cit.*

Suitable pharmaceutically acceptable *in vivo* hydrolysable esters include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic, and alkanedioic acids, in which each alkyl or alkenyl moiety advantageously has not more than 6 carbon atoms. Examples of particular esters include acetates, propionates, butyrates, acrylates, and ethylsuccinates.

The present invention also includes acid addition salts, especially pharmaceutically acceptable acid addition salts, of the compounds of the general formula I. Such acid addition salts may, in particular, be formed at the 3'-dimethylamino group of the desosamine ring.

Various acid addition salts of erythromycin are described in US 2 761 859 (C.E. Hoffhine, Jr.; Abbott Laboratories) and US 2 852 429 (J.T. Shepler; Eli Lilly).

Suitable acid addition salts of the compounds of the invention include pharmaceutically acceptable inorganic

acid addition salts, for example the sulphate, nitrate, phosphate, borate, hydrochloride and hydrobromide, and also pharmaceutically acceptable organic acid addition salts, for example the acetate, tartrate, maleate, citrate, succinate, benzoate, ascorbate, methane-sulphate, α-keto-glutarate, α-glycerophosphate, and glucose-1-phosphate. Preferably the acid addition salt is the laurylsulphate salt.

Examples of individual compounds according to the present invention include:

(i) 6-O-methylerythromycin A 9-methoxime (in general formula I, $R^1 + R^2 = {=}N{-}OCH_3$, $R^3 = CH_3$, $R^4 = OH$, $R^5 = H$, $R^7 = CH_3$, $R^8 = H$, $R^9 = OH$); and

(ii) 6-O-ethylerythromycin A 9-methoxime (in general formula I, $R^3 = C_2H_5$, $R^1 + R^2$ and $R^4$ to $R^9$ as for compound (i));

as well as

corresponding 11-ether derivatives, and

corresponding derivatives in which the 4''-position is modified as discussed above;

and also

pharmaceutically acceptable esters and acid addition salts of such compounds.

The 6-ether erythromycin 9-oxime ether derivatives according to the invention may be prepared by reacting an erythromycin 9-oxime or 9-oxime ether derivative

having a hydroxy substituent at the 6-position, in which any reactive groups (other than the 6-hydroxy group) may optionally be protected, with a alkylating agent; and thereafter if necessary carrying out one or more of the following steps:

(a) converting a substitutent on the erythromycin structure to another such substituent in a conventional manner;

(b) removing any protecting groups; and

(c) forming a pharmaceutically acceptable ester or acid addition salt.

The resulting 9-oxime ether compound according to the invention may, if desired, subsequently be converted to a 9-oxime compound according to the invention, which may, if desired, be converted to a 9-imino compound according to the invention, which in turn may, if desired, be converted into a 9-amino compound according to the invention, which may, if desired, be further converted to a 9-substituted amino compound according to the invention.

More particularly, a compound of the general formula I as hereinbefore defined or a pharmaceutically acceptable ester or acid addition salt thereof may be prepared by a process which comprises reacting a compound of the general formula VI:

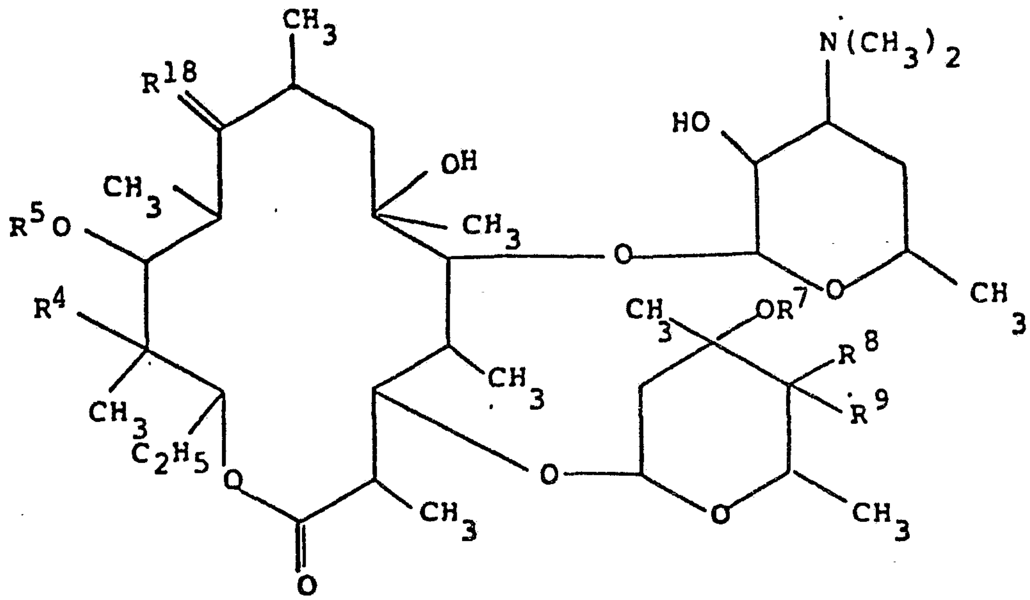

VI

wherein

$R^4$, $R^5$, $R^7$, $R^8$ and $R^9$ are defined as above with respect to general formula I,and

$R^{18}$ denotes an oxime or oxime ether group,

in which compound of the general formula VI any reactive group (other than the 6-hydroxy group) may optionally be protected,

with an alkylating agent,

to give a compound of the general formula I in which $R^1$ and $R^2$ together denote an oxime ether group;

and thereafter, if necessary or desired, carrying out one or more of the following steps in any suitable order:

(a) converting an oxime ether group denoted by $R^1$ and $R^2$ together to another oxime ether group or an oxime group;

(b) converting a resulting oxime group denoted by $R^1$ and $R^2$ together to an oxime ether group or an imino group;

(c) converting a resulting imino group denoted by $R^1$ and $R^2$ together to an amino group denoted by $R^1$ or $R^2$;

(d) converting a resulting amino group denoted by $R^1$ or $R^2$ to a substituted amino group;

(e) converting any one or more of the groups denoted by $R^5$, $R^8$ and $R^9$ to another such group;

(f) removing any protecting group that may be present; and

(g) forming a pharmaceutically acceptable ester or acid addition salt.

The compound of the general formula VI in which:

each of $R^4$ and $R^9$ denotes hydroxy,

each of $R^5$ and $R^8$ denotes hydrogen, and

$R^7$ denotes methyl

is erythromycin A 9-oxime or a 9-oxime ether thereof, which may be prepared from erythromycin A by known methods, for example by the methods described in the

above-cited references relating to erythromycin 9-oximes and 9-oxime ethers.

The compound of the general formula VI in which:

each of $R^4$, $R^5$ and $R^8$ denotes hydrogen, and

$R^7$ denotes methyl, and

$R^9$ denotes hydroxy

is erythromycin B 9-oxime or a 9-oxime ether thereof and may be prepared from erythromycin B by analogous known methods.

Other compounds of the general formula VI may also be prepared, by methods known *per se*, from erythromycin A or B or the corresponding 9-oxime or 9-oxime ether derivative. For example, a compound in which the 4''-position is substituted other than as in naturally-occuring erythromycin A or B (that is to say, in which $R^8$ is other than hydrogen and/or $R^9$ is other than hydroxy) may be prepared as described in the respective references cited above.

In general, in the preparation of compounds of the general formula VI, the conversion of the 9-oxo group of erythromycin A or B to a 9-oxime or 9-oxime ether group may be effected prior to or subsequent to modification of other positions of the erythromycin molecule.

Prior to carrying out the reaction of a compound of the general formula VI with an alkylating agent, any reactive group of a compound of the general formula VI

(other than the 6-hydroxy group) may optionally be protected.

In particular, the 3'-dimethylamino group will generally be protected by an N-protecting group. The N-protection may be effected in known manner, for example by the method described by E.H. Flynn et al, (J. Amer. Chem. Soc., 1955, 77, 3104-3106).

Examples of suitable N-protecting groups include benzyloxycarbonyl, and substituted benzyloxycarbonyl, (for example, p-methylbenzyloxycarbonyl, p-nitrobenzyloxycarbonyl, p-bromobenzyloxycarbonyl, p-phenylazobenzyloxycarbonyl, and p-(p'-methoxy-phenylazo)-benzyloxycarbonyl). A preferred N-protecting group is benzyloxycarbonyl.

It may also be advantageous to protect one or more of the hydroxy groups present in the erythromycin molecule (other than the 6-hydroxy group) prior to reaction. In particular, it may be advantageous to protect any hydroxy groups present at the 2'- and 4''-positions, especially the 2'-hydroxy group. It is convenient to employ the same group to protect the hydroxy group(s) as that employed to protect the amino moiety, especially a benzyloxycarbonyl group.

Any reactive substituents that may be present in the group $R^8$ or $R^9$ should preferably also be protected in a conventional manner.

The present invention also provides compounds of the general formula VII, which are useful as intermediates in the preparation of the compounds of the general formula I:

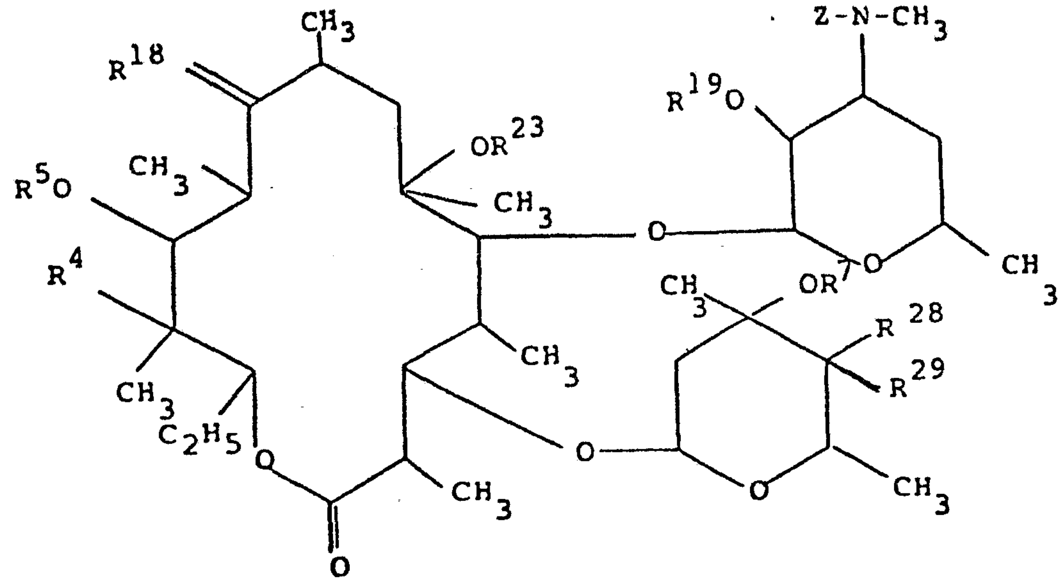

VII

in which

$R^4$, $R^5$, $R^7$ and $R^{18}$ are defined as above;

$R^{19}$ denotes H or Z;

$R^{23}$ denotes H or $R^3$ (where $R^3$ is defined as above), with the proviso that, if $R^{18}$ denotes methoxime and $R^5$ denotes hydrogen, then $R^{23}$ cannot denote methyl;

one of $R^{28}$ and $R^{29}$ denotes H, OH, OZ, $NZ_2$, $NH_2$, NHZ, substituted $NH_2$, substituted NHZ, alkoxy, alkanoyloxy, or $R^{12}-SO_2-O-$ (in which $R^{12}$ denotes an organic group), and the other of $R^{28}$ and $R^{29}$ denotes H, or

$R^{28}$ and $R^{29}$ together denote oxo; and

Z denotes a protecting group, more particularly an N-protecting group, preferably a substituted benzyloxycarbonyl group or, especially, a benzyloxycarbonyl group.

In the process according to the invention, the erythromycin compound of the general formula VI, optionally containing protective groups, is reacted with an alkylating agent, that is to say, a reagent that will react with the 6-hydroxy group to form a 6-alkyl ether group (including a 6-(substituted alkyl)ether group), $-OR^3$, suitably in the presence of a strong base and an organic solvent.

The alkylating agent may be any suitable known alkylating agent, for example an alkyl iodide (for example, methyl iodide or ethyl iodide), a dialkyl sulphate (for example, dimethyl sulphate), or an alkyl *p*-toluene-sulphonate (for example, methyl *p*-toluenesulphonate).

The alkylating agent may be represented by the general formula VIII:

$$R^3 - X \qquad\qquad VIII$$

in which

$R^3$ is defined as above, and

X denotes a leaving group.

Examples of suitable leaving groups X include halogen atoms (for example chlorine, bromine, and iodine), alkylsulphonyloxy groups (for example methanesulphonyloxy), and arylsulphonyloxy groups (for example p-toluenesulphonyloxy). Preferably, X denotes a halogen atom, especially an iodine atom.

In the process according to the invention, the reaction of the erythromycin compound of the general formula VI

with the alkylating agent is suitably carried out under strongly basic conditions. Examples of suitable strong bases include alkali metal hydrides (for example, sodium hydride or potassium hydride), alkali metal amides (for example, lithium amide, sodium amide, potassium amide, or lithium diisopropylamide), alkali metal alkanoates (for example, potassium t-butoxide), and organometallic compounds (for example, butyllithium).

If the group $R^3$ contains any reactive substituents, that is to say, substituents that will react under the above-mentioned conditions, (including, for example, some of the substituents mentioned previously for the group $R^3$), such substituents may advantageously be protected in conventional manner prior to reaction of the alkylating agent with the erythromycin compound of the general formula VI.

The alkylation reaction may suitably be carried out in an inert solvent. Suitable solvents include, for example, N,N-dimethylformamide, N,N-dimethylacetamide, dimethylsulphoxide, hexamethylphosphoric triamide, N-methylpyrrolidinone, tetrahydrofuran, dioxan, ethoxyethane, and 1,2-dimethoxyethane, and also mixtures of two or more such solvents.

The alkylation reaction may suitably be carried out using from 5 to 10 moles of the alkylating agent and from 1 to 2 moles of the base per mole of the erythromycin compound of the general formula VI.

The said reaction may suitably be effected at a cool to ambient temperature preferably at a cool temperature. The reaction may, for example, be effected at a temperature within the range of from -30°C to +30°C,

preferably from -10°C to +20°C, especially from -5°C to +5°C.

If the initial erythromycin compound of the general formula VI contains a 9-oxime group, that group will react with the alkylating agent to form a 9-oxime ether. Alternatively, the 9-oxime group may first be protected by means of an ether group, so that the alkylation reaction is in fact carried out on a 9-oxime ether. Such protection may be effected in known manner using known ether-forming hydroxy-protecting groups, for example benzyl, substituted benzyl, tetrahydropyranyl, and trichlorethyl groups.

In any event, the erythromycin compound resulting from the alkylation reaction contains a 9-oxime ether group. If the desired product of the general formula I contains such a group, no reaction need be carried out at the 9-position, although a 9-oxime ether group may optionally now be converted into a 9-oxime group or into another 9-oxime ether group. If the desired product of the general formula I contains a 9-imino group, the 9-oxime group obtained by conversion from a 9-oxime ether group may next be converted to a 9-imino group and that in turn may, if necessary, be converted to a 9-(optionally substituted)-amino group.

All such conversions at the 9-position may be carried out in known manner, for example as described in the above-cited references. For example, the oxime may be converted to the imine by reaction with titanium trichloride in known manner, and the imine may be converted to the amine by reaction with sodium borohydride in known manner.

Also after completion of the alkylation reaction, and prior or subsequent to any conversion of the 9-substituent, any of the groups $R^5$, $R^8$ and $R^9$ may be converted to any of the other such groups within the definitions given above by methods known in the art, for example by the methods disclosed in the above-cited references. For example, a compound in which $R^9$ denotes hydrogen and $R^8$ denotes hydroxy can be converted to a compound in which $R^8$ and $R^9$ together denote oxo and optionally thereafter to a compound in which $R^9$ denotes hydroxy or acetoxy and $R^8$ denotes hydrogen by methods analogous to those described in US 3 884 903, op. cit..

After completion of the alkylation reaction, any protecting groups may be removed by a conventional method. It is often appropriate to employ a hydrogenation procedure.

The hydrogenation may suitably be carried out in the presence of a transition metal catalyst, for example palladium, which may, for example, be in the form of palladium on carbon (charcoal), palladium on barium sulphate, palladium on calcium carbonate, or palladium black. A favoured catalyst is palladium on carbon (sometimes referred to as palladium on charcoal); for example 5%, 10%, 20% or 30% palladium on carbon. A low, medium or high pressure of hydrogen may be used in this reaction, for example a pressure of from 1 to 6 atmospheres absolute, a pressure of 1 atmosphere absolute being convenient. The reaction may suitably be carried out at a non-extreme temperature, for example at a temperature within the range of from 0°C to 30°C, preferably from 12°C to 25°C. It is generally convenient to carry out the reaction at ambient temperature. The reaction is preferably

carried out at a pH within the range of from 4.5 to 5.0, which may be maintained by the use of a suitable buffer, for example an acetate buffer at pH 4.8. Suitable solvents for carrying out the hydrogenation include ethanol, n-propanol, isopropanol, tetrahydrofuran, dioxan, ethyl acetate, a mixture of two or more such solvents, or such a solvent or mixture in the presence of water. A favoured solvent is ethanol.

In order to restore the dimethylamino group at the 3'-position, it is convenient to effect a reductive methylation, which advantageously may be carried out at the same time as the reductive removal of the protecting groups, as in the method of Flynn et al, op. cit..

A compound of the general formula I may be converted to a pharmaceutically acceptable salt thereof or ester thereof in a conventional manner at any convenient stage in the manufacturing process, for example before or after the removal of any protecting groups and/or before or after any conversion of the 9-substituent and/or of groups $R^5$, $R^8$ and $R^9$ to other such groups.

Isolation and purification of a compound according to the invention may be carried out using conventional methods, and may include a chromatography step. Preferably the product is isolated in crystalline form.

The compounds according to the invention, that is to say, the compounds of the general formula I and their pharmaceutically acceptable salts and esters, have antibacterial properties and are useful for the treatment of bacterial infections in animals, especially mammals, including humans, in particular

0194833



humans and domesticated animals (including farm animals). The compounds may be used for the treatment of infections caused by a wide range of gram-positive and gram-negative organisms including, for example, *Bacillus subtilis*, *Corynebacterium xerosis*, *Sarcina lutea*, *Staphylococcus aureus*, *Streptococcus faecalis*, *Streptococcus pyogenes*, *Streptococcus agalactiae*, *Streptococcus pneumoniae*, *Haemophilus sp*. *Neisseria sp*., *Chlamydia sp*., and *Legionella sp*..

The present invention provides a pharmaceutical composition comprising a compound according to the invention together with a pharmaceutically acceptable carrier or excipient.

The present invention also provides a method of treating bacterial infections in animals, especially in humans and in domesticated mammals, which comprises administering a compound or composition according to the invention to a patient in need thereof.

The compounds and compositions according to the invention may be formulated for administration in any convenient way for use in human or veterinary medicine, by analogy with other antibiotics.

The compounds and compositions according to the invention may be formulated for administration by any route, for example oral, topical or parenteral. The compositions may, for example, be made up in the form of tablets, capsules, powders, granules, lozenges, creams, syrups, or liquid preparations, for example solutions or suspensions, which may be formulated for oral use or in sterile form for parenteral administration by injection or infusion.

Tablets and capsules for oral administration may be in unit dosage form, and may contain conventional excipients including, for example, binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or another suitable vehicle before use. Such liquid preparations may contain conventional additives, including, for example, suspending agents, for example sorbitol, methyl cellulose, glucose syrup, gelatin, hydroxyethyl cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and colouring agents.

A compound or composition according to the invention may suitably be administered to the patient in an antibacterially effective amount. A composition according to the invention may suitably contain from

0.1% by weight, preferably from 10 to 60% by weight, of a compound according to the invention (based on the total weight of the composition), depending on the method of administration.

The compounds according to the invention may suitably be administered to the patient at a daily dosage of from 1.5 to 50 mg/kg of body weight. For an adult human (of approximately 70 kg body weight), from 100 to 3000 mg, for example about 1500 mg, of a compound according to the invention may be administered daily. Suitably, the dosage for adult humans is from 5 to 20 mg/kg per day. Higher or lower dosages may, however, be used in accordance with normal clinical practice.

When the compositions according to the invention are presented in unit dosage form, each unit dose may suitably comprise from 25 to 1000 mg, preferably from 50 to 500 mg, of a compound according to the invention.

No adverse toxicological effects are indicated when the compounds according to the invention are administered within the above-mentioned dosage ranges.

The following examples illustrate the preparation of compounds according to the present invention.

Example 1

6-O-Methylerythromycin A 9-methoxime

a) 6-O-Methyl-2'-O,N-dibenzyloxycarbonyl-des-N-methylerythromycin A 9-methoxime

2'-O,N-Dibenzyloxycarbonyl-des-N-methylerythromycin A 9-oxime (500 mg) in dry N,N-dimethylformamide (8 ml) was treated with methyl iodide (0.5 ml) and the mixture was stirred and ice-cooled while sodium hydride (50% dispersion in oil; 50 mg) was added in one portion. The mixture was stirred and ice-cooled for 30 min, and was then diluted with ethyl acetate (60 ml). The solution was washed with dilute sodium sulphite and water (3 x). The solution was dried ($Na_2SO_4$) and the solvent was removed under reduced pressure to yield a colourless gum. Chromatography on silica gel using 1:3 to 2:3 ethyl acetate/hexane gave the title compound as a colourless gum (240 mg); (Found: $M^+$, 1030.5642; $C_{54}H_{82}N_2O_{17}$ requires M, 1030.5617).

b) 6-O-Methylerythromycin A 9-methoxime

6-O-Methyl-2'-O,N-dibenzyloxycarbonyl-des-N-methylerythromycin A 9-methoxime (220 mg) in ethanol (12 ml) and acetate buffer (pH 4.8; 1 ml) was shaken with 10% palladium-charcoal (70 mg) under hydrogen (1 atmosphere) for 30 min. 37% Formaldehyde (1 ml) was added and hydrogenation was continued for 1.5 h. The catalyst was removed by filtration and was washed with ethanol and water. The ethanol was removed from the filtrate under reduced pressure and the aqueous residue was brought to pH 12 by addition of potassium carbonate. The mixture was extracted with ethyl

acetate (2 x) and the organic solution was washed with water and dried ($Na_2SO_4$). The solvent was removed under reduced pressure to give the title compound as a colourless foam (170 mg); (Found: $M^+$, 776.5030; $C_{39}H_{72}N_2O_{13}$ requires M, 776.5038).

Example 2

6-O-Ethylerythromycin A 9-methoxime

a) 6-O-Ethyl-2'-O,N-dibenzyloxycarbonyl-des-N-methylerythromycin A 9-methoxime

2'-O,N-dibenzyloxycarbonyl-des-N-methylerythromycin A 9-methoxime (200 mg) in N,N-dimethylformamide (10 ml) was treated with ethyl iodide (1 ml) and the mixture was stirred and ice-cooled while sodium hydride (50% dispersion in oil; 9.5 mg) was added in one portion. The mixture was stirred and ice-cooled for 45 min., and was then diluted with ethyl acetate and washed with dilute sodium sulphite and water (3 x). The solution was dried ($MgSO_4$) and the solvent was evaporated under reduced pressure to yield a colourless gum. Chromatography on silca gel using 1:1 ethyl acetate/hexane gave the title compound as a colourless gum (73 mg); (Found: $M^+$, 1044.5753; $C_{55}H_{84}N_2O_{17}$ requires M, 1044.5774).

b) 6-O-Ethylerythromycin A 9-methoxime

6-O-Ethyl-2'-O,N-dibenzyloxycarbonyl-des-N-methylerythromycin A 9-methoxime (130 mg) was converted into the title compound using the process described in Example 1(b). The title compound was obtained as a colourless foam (76 mg), $[\alpha]^{20}_D = -79.4^o$ (conc. 1% in $CHCl_3$).

Claims

1. A compound of the general formula I or a pharmaceutically acceptable ester or acid addition salt thereof:

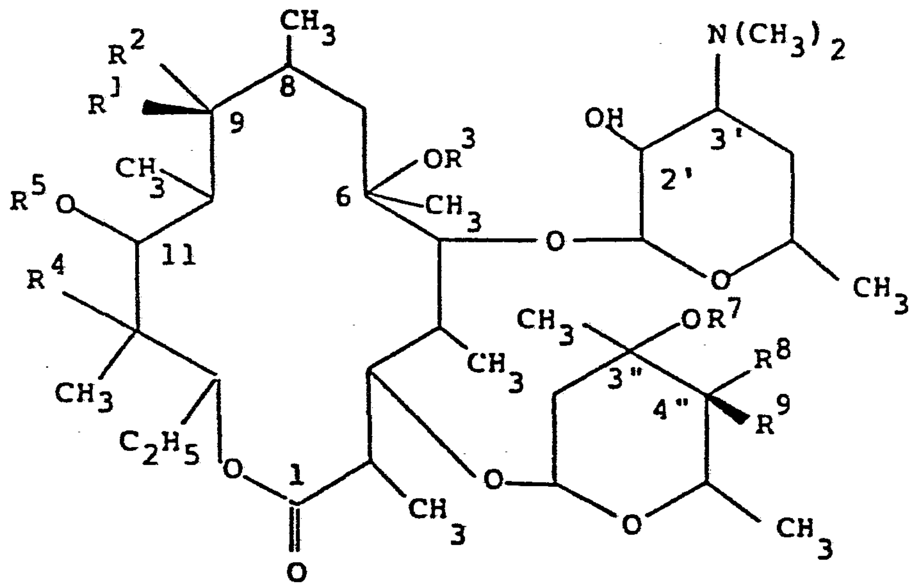

I

wherein

one of $R^1$ and $R^2$ denotes hydrogen and the other of $R^1$ and $R^2$ denotes an amino group or a substituted amino group, or

$R^1$ and $R^2$ together denote an oxime group, an oxime ether group, or an imino group;

$R^3$ denotes an unsubstituted or substituted alkyl group;

$R^4$ denotes hydrogen or hydroxy;

$R^5$ denotes hydrogen, an alkyl group, or a group of the formula $-CHR^{10}SR^{11}$;

$R^7$ denotes hydrogen or methyl;

one of $R^8$ and $R^9$ denotes hydrogen, hydroxy, alkoxy, alkanoyloxy, amino, substituted amino, or a group of the formula $R^{12}$-$SO_2$-O-, and the other of $R^8$ and $R^9$ denotes hydrogen, or

$R^8$ and $R^9$ together denote an oxo group, an oxime group, or a substituted oxime group;

$R^{10}$ denotes hydrogen or an alkyl group;

$R^{11}$ denotes an alkyl group; and

$R^{12}$ denotes an organic group.

2. A compound as claimed in claim 1, wherein $R^3$ denotes an unsubstituted or substituted primary $(C_{1-6})$alkyl group, especially a methyl group.

3. A compound as claimed in claim 1 or claim 2, wherein $R^1$ and $R^2$ together denote a group of the formula IV:

$$=N-O-R^{13} \qquad \text{IV}$$

in which $R^{13}$ denotes hydrogen or an unsubstituted or substituted hydrocarbon group especially a hydrogen atom or a methyl group.

4. A compound as claimed in any one of claims 1 to 3, wherein $R^4$ denotes a hydroxy group and $R^7$ denotes a methyl group.

5. A compound as claimed in any one of claims 1 to 4, wherein $R^5$ denotes a hydrogen atom.

6. A compound as claimed in any one of claims 1 to 5, wherein $R^8$ denotes a hydrogen atom and $R^9$ denotes a hydroxy group.

7. The compounds

(i) 6-O-methylerythromycin A 9-methoxime; and

(ii) 6-O-ethylerythromycin A 9-methoxime;

corresponding 11-ether derivatives, and

corresponding derivatives in which the 4''-position is modified; and

pharmaceutically acceptable esters and acid addition salts of such compounds.

8. A process for the preparation of a compound of the general formula I defined in claim 1 or a pharmaceutically acceptable ester or acid addition salt thereof, which comprises reacting a compound of the general formula VI:

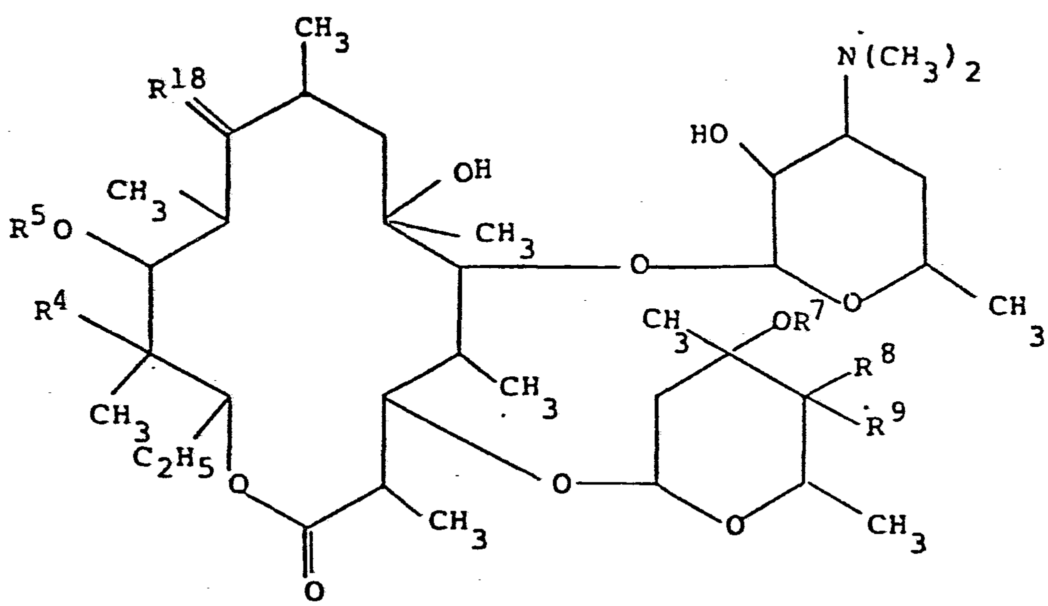

VI

wherein

$R^4$, $R^5$, $R^7$, $R^8$ and $R^9$ are defined as in claim 1,

$R^{18}$ denotes an oxime or oxime ether group,

in which compound of the general formula VI any reactive group (other than the 6-hydroxy group) may optionally be protected,

with an alkylating agent,

to give a compound of the general formula I in which $R^1$ and $R^2$ together denote an oxime ether group;

and thereafter, if necessary or desired, carrying out one or more of the following steps in any suitable order:

(a) converting a substitutent on the erythromycin structure to another such substituent in a conventional manner;

(b) removing any protecting groups; and

(c) forming a pharmaceutically acceptable ester or acid addition salt.

9. A process for the preparation of a compound of the general formula I defined in claim 1 in which $R^3$ denotes a methyl group, or a pharmaceutically acceptable ester or acid addition salt thereof, which comprises reacting a compound of the general formula VI:

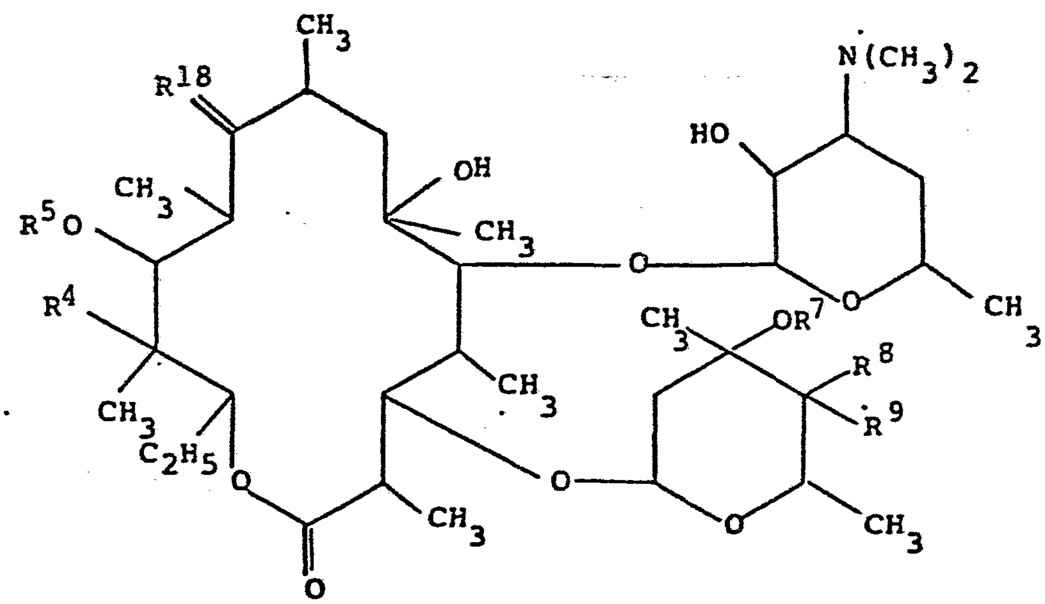

VI

wherein

$R^4$, $R^5$, $R^7$, $R^8$ and $R^9$ are defined as in claim 1,

$R^{18}$ denotes an oxime or oxime ether group,

in which compound of the general formula VI the 2′-hydroxy group and/or the 3′-dimethylamino group is/are protected and any other reactive group (other than the 6-hydroxy group) may optionally be protected,

with a methylating agent,

to give a compound of the general formula I in which $R^1$ and $R^2$ together denote an oxime ether group, and $R^3$ denotes a methyl group;

and thereafter, if necessary or desired, carrying out one or more of the following steps in any suitable order:

(a) converting a substitutent on the erythromycin structure to another such substituent in a conventional manner;

(b) removing any protecting groups; and

(c) forming a pharmaceutically acceptable ester or acid addition salt.

10. A pharmaceutical composition which comprises a compound as claimed in any one of claims 1 to 7 in admixture or conjunction with a pharmaceutically acceptable carrier or excipient.

11. The use of a compound as claimed in any one of claims 1 to 7 for the manufacture of a medicament.